# EUROPÄISCHE PATENTSCHRIFT

(11) **EP 0 404 716 B1**
(45) Veröffentlichungstag und Bekanntmachung des Hinweises auf die Patenterteilung: **20.01.1993**
(21) Anmeldenummer: 90810336.9
(22) Anmeldetag: 03.05.1990
(51) Int. Cl.: A61F 2/36

(54) **Verankerungsschaft für eine Femurkopfprothese**
Anchored shaft for a femoral prosthesis
Tige d'ancrage pour une prothèse fémorale

(30) Priorität: 21.06.1989 CH 2316/89
(43) Veröffentlichungstag der Anmeldung: 27.12.1990
(73) Patentinhaber: GEBRÜDER SULZER AKTIENGESELLSCHAFT, CH-8401 Winterthur (CH); PROTEK AG, 3110 Münsingen-Bern (CH)
(72) Erfinder: Spotorno, Lorenzo, Prof.-Dr.-med., I-17024 Finale Ligure (IT); Frey, Otto, Dr., CH-8400 Winterthur (CH)

(56) Entgegenhaltungen:
- EP-A- 0 220 803
- EP-A- 0 308 297
- EP-A- 0 359 672
- US-A- 4 187 559

## Beschreibung

Die Erfindung betrifft einen Verankerungsschaft für eine Femurkopfprothese, bei der zwischen dem in den Femur implantierbaren Schaft und dem den Gelenkkopf tragenden Prothesenhals zur Abstützung auf dem Femurknochen ein Kragen angeordnet ist. Ein derartiger Verankerungsschaft ist aus der EP-A-0 308 297 bekannt.

Eine proximale Weiterleitung der Druckbelastungen von einer Prothese auf den Femurknochen erfolgt sehr häufig über den Kragen der Prothese, der den Prothesenhals von dem eigentlichen Schaft trennt und sich für eine derartige Kraftübertragung auf den kortikalen Rand der Resektionsebene abstützen muss.

Es hat sich nun gezeigt, dass es infolge der Belastungsänderungen am Gelenkkopf zu Wechselbelastungen zwischen Kragen und kortikalem Knochengewebe kommt, die im Laufe der Zeit zum Abbau des Knochens und damit mindestens zu einer Verminderung der maximalen Krafteinleitung in den Knochen führt.

Aufgabe der Erfindung ist es daher, einen Verankerungsschaft zu schaffen, bei dem ein inniger und möglichst weitgehend konstanter Kontakt des Kragens mit dem Knochen möglichst bei allen wechselnden Belastungen, die auf den Gelenkkopf der Prothese wirken, gewährleistet ist. Diese Aufgabe wird mit der Erfindung dadurch gelöst, dass der Schaft medial unterhalb des und nahe dem Kragen einen Schlitz aufweist, der sich im Schaftkörper zu einer blasenförmigen Aussparung erweitert, und dass ferner mindestens die Aussparung mit einem Elastomer-Füllkörper gefüllt sind.

Bei ordnungsgemässer Implantation liegt der Kragen vor allem in seinem medialen Bereich bei der neuen Konstruktion unter einer Vorspannung auf dem Knochengewebe auf, da sich der Schlitz und die Aussparung aufgrund der Elastizität der lateral der Aussparung gelegenen brückenartigen Verbindung vom distalen Schaftteil zum Prothesenhals bzw. Prothesenkragen maulartig elastisch öffnet. Der Füllkörper in der Aussparung hat eine hohe Elastizität, damit er die durch die laterale Verbindung des Schaftkörpers bestimmte Elastizität im proximalen Schaftbereich nicht begrenzt oder einengt. Die Tiefe der Aussparung in lateraler Richtung und damit bei gegebener Schaftform die Querschnittsfläche der lateralen Verbindung können dabei mit Vorteil so gewählt werden, dass auf der einen Seite die notwendige Schaftfestigkeit gewährleistet ist, auf der anderen Seite jedoch die "Elastizität" des Kragens bei den wechselnden Belastungen mindestens weitgehend der Elastizität des kortikalen Knochenmaterials entspricht, so dass Knochen und Kragen bei ihr elastischen "Schwingungen" infolge der Be- und Entlastungen möglichst gleiche Wege zurücklegen. Dadurch kann die Konstanz des Kragenskontaktes mit dem Knochen verbessert werden. Infolge seiner grossen Elastizität weicht der Füllkörper darüberhinaus bei Druckbelastungen mit seinem verdrängten Volumen in Richtungen quer zur Längsachse des Schaftes aus, wodurch seine Anpressung an die Wand des Operationshohlraumes vergrössert wird. Eine weitere Aufgabe des Füllkörpers besteht darin, ein Durch- und Einwachsen von Gewebe in die Ausparung zu verhindern.

Als Material für den Füllkörper haben sich Silikone und Polyurethane mit Shore A-Härten von 50 bis 80 und Shore D-Härten von 30 bis 60 als besonders geeignet erwiesen; weiterhin hat sich gezeigt, dass am medialen Rand Schlitzbreiten von 2 bis 4 mm einen ausreichenden Spielraum für eine Bewegung des Kragens bei Druckbelastungen gewährleisten.

Im folgenden wird die Erfindung anhand eines Ausführungsbeispiels im Zusammenhang mit der Zeichnung näher erläutert.
Fig. 1 zeigt eine Ansicht des neuen Schaftes von anterior oder posterior her;
Fig. 2 gibt eine Ansicht des proximalen Bereichs von Fig. 1 von rechts wieder, während
Fig. 3 und 4 die Schnitte III-III und IV-IV darstellen.

Der in dem gezeigten Beispiel als Geradschaft ausgebildete Schaft 1, der aus Metall, beispielsweise einer Titanlegierung besteht, erweitert sich von seinem distalen Ende 2 allseitig konisch. Seine laterale Schmalseite 3 geht proximal über einen Kreisbogen zunächst in die Horizontale über und mündet in einen Prothesenhals 4; dieser trägt seinerseits einen konischen Zapfen 5, auf den ein nicht dargestellter Gelenkkopf aufsteckbar ist.

Aus dem sich erweiternden Konus heraus verläuft die mediale Schmalseite 6 des Schaftes in einer stetig gekrümmter Kurve und endet an einem Kragen 7, der den Prothesenhals 4 von dem eigentlichen Schaftkörper 1 trennt. Die Grundfläche des Kragens 7, die z.B. senkrecht zur Achse 8 des Prothesenhalses 4 ausgerichtet sein kann, bildet eine relativ grosse Abstützfläche, mit der sich der Kragen 7 auf dem koritikalen Gewebe des nicht dargestellten Femurknochens abstützt.

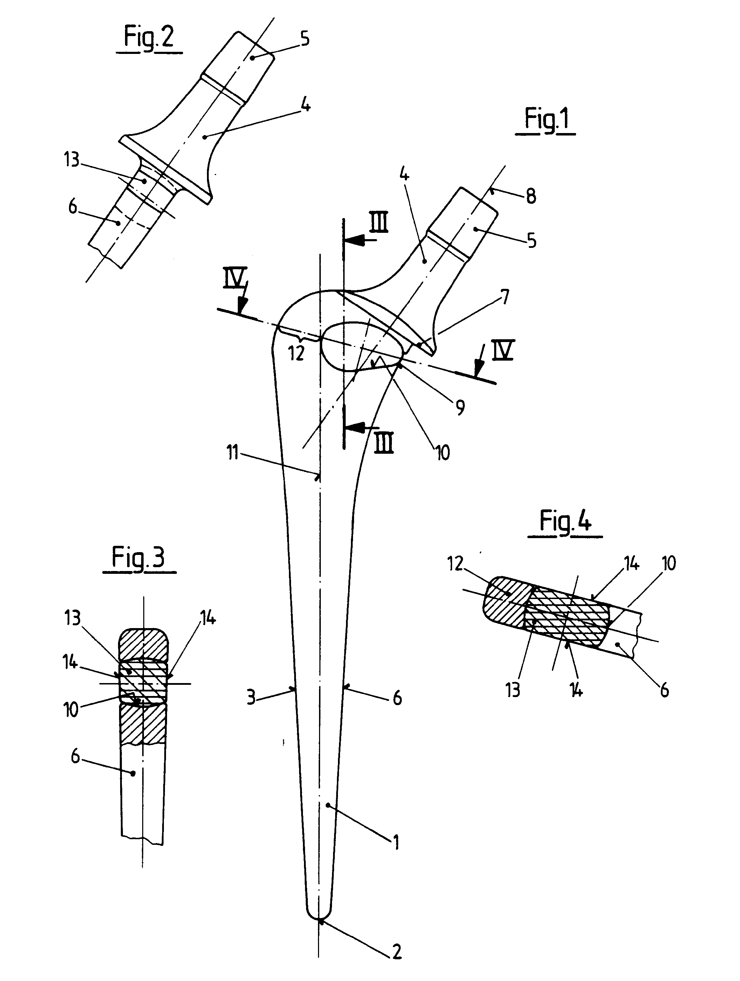

Wenige Millimeter unterhalb des Kragens 7 weist die mediale Schmalseite 6 einen Schlitz 9 auf, der sich nach lateral zu einer blasenartigen Aussparung 10 im Schaftkörper 1 erweitert. In dem gezeigten Beispiel erstreckt sich die Aussparung 10 in Richtung auf den grossen Trochanter bis etwa zur Längsmittelebene 11 des Geradschaftes 1. Jenseits von dieser verbindet ein brückenartiger Bogen 12 den distalen Teil des Schaftes 1 mit dem Kragen 7 bzw. dem Prothesenhals 4. Wie Fig. 4 zeigt, weist der "Brückenbogen" 12 einen im wesentlichen quadratischen Querschnitt auf. Seine Form und seine Abmessungen sind einerseits durch die geforderte Mindestfestigkeit für den Schaft 1 und andererseits - wie bereits geschildert - durch die gewünschte "Elastizität" des Kragens 7 bestimmt, die an diejenige des kortikalen Knochengewebes angeglichen sein soll.

Ausgefüllt wird die Aussparung 10 von einem hochelastischen Füllkörper 13, der im vorliegenden Beispiel aus Polyurethan besteht. Er kann an seinen, sich im Knochen abstützenden Oberflächen 14 mit einer, das Anwachsen von Gewebe fördernden Beschichtung, beispielsweise aus Hydroxylapatit belegt sein. Ebenso kann man die Grundfläche des Kragens 7, die auf dem Knochen zur Auflage kommt, mit einer, ein Anwachsen des Knochens fördernden Struktur versehen sein.

## Patentansprüche

1. Verankerungsschaft für eine Femurkopfprothese, bei der zwischen dem in den Femur implantierbaren Schaft (1) und dem den Gelenkkopf tragenden Prothesenhals (4) zur Abstützung auf dem Femurknochen ein Kragen (7) angeordnet ist, dadurch gekennzeichnet, dass der Schaft (1) medial unterhalb des Kragens (7) und nahe dem Kragen (7) einen Schlitz (9) aufweist, der sich im Schaftkörper (1) zu einer blasenförmigen Aussparung (10) erweitert, und dass ferner mindestens die Aussparung (10) mit einem Elastomer-Füllkörper (13) gefüllt ist.

2. Verankerungsschaft nach Anspruch 1, dadurch gekennzeichnet, dass der Schlitz (9) am medialen Rand (6) des Schaftes (1) eine Breite von 2 bis 4 mm hat.

3. Verankerungsschaft nach Anspruch 1 oder 2, dadurch gekennzeichnet, dass der Füllkörper (13) aus Polyurethan besteht.

4. Verankerungsschaft nach Anspruch 1 oder 2, dadurch gekennzeichnet, dass der Füllkörper (13) aus einem Silikon besteht.

5. Verankerungsschaft nach einem der Ansprüche 1 bis 4, dadurch gekennzeichnet, dass die Erstreckung der Aussparung (10) nach lateral durch die geforderte Elastizität und/oder Festigkeit des Schaftes (1) bestimmt ist.

## Claims

1. An anchoring stem for a femur head prosthesis, in which between the stem (1) for implantation in the femur and the prosthesis neck (4) bearing the joint head a collar (7) is mounted for support on the femur bone, characterised in that the stem (1) has medially below the collar (7) and near the collar (7) a slot (9) which widens in the stem body (1) to form a pocket-like recess (10), and in addition at least the recess (10) is filled with an elastomeric packing member (13).

2. An anchoring stem as claimed in claim 1, characterised in that the slot (9) has a width of 2 to 4 mm at the medial edge (6) of the stem (1).

3. An anchoring stem as claimed in claim 1 or 2, characterised in that the packing member (13) is of polyurethane.

4. An anchoring stem as claimed in claim 1 or 2, characterised in that the packing member (13) is of silicone.

5. An anchoring stem as claimed in any of claims 1 to 4, characterised in that the dimension of the recess (10) in the lateral direction is determined by the required resilience and/or strength of the stem (1).

## Revendications

1. Tige d'ancrage d'une prothèse de tête de fémur, dans laquelle un collet (7), destiné à prendre appui sur le fémur, est placé entre la tige (1) à implanter dans le fémur et le col de prothèse (4) portant la tête d'articulation, caractérisée en ce que la tige (1) présente dans la région médiale, au-dessous du collet (7) et à proximité de celui-ci, une fente (9) qui s'élargit dans le corps de tige (1) pour former une découpe (10) en forme de bulle et en ce qu'en outre la découpe (10) au moins est remplie d'une garniture en élastomère (13).

2. Tige d'ancrage selon la revendication 1, caractérisée en ce que la fente (9) présente sur le bord médial (6) de la tige (1) une largeur comprise entre 2 et 4 mm.

3. Tige d'ancrage selon la revendication 1 ou 2, caractérisée en ce que la garniture (13) est en polyuréthanne.

4. Tige d'ancrage selon la revendication 1 ou 2, caractérisée en ce que la garniture (13) est en une silicone.

5. Tige d'ancrage selon l'une des revendications 1 à 4, caractérisée en ce que l'extension de la découpe (10) vers la région latérale est déterminée par l'élasticité et/ou la résistance voulue de la tige (1).
